# EUROPEAN PATENT SPECIFICATION

(11) **EP 2 971 232 B1**
(45) Date of publication and mention of the grant of the patent: **09.09.2026**
(21) Application number: 14721031.4
(22) Date of filing: 10.03.2014
(51) Int. Cl.: C23C 18/16, C23C 18/18, C23C 18/54, B06B 1/06, H10N 30/06

(54) **PLATING METHOD FOR COATING ULTRASOUND TRANSDUCERS**
VERFAHREN ZUM BESCHICHTEN VON ULTRASCHALLWANDLERN
PROCÉDÉ DE PLACAGE POUR REVETIR DES TRANSDUCTEURS À ULTRASONS

(30) Priority: 14.03.2013 US 201361784164 P
(43) Date of publication of application: 20.01.2016
(73) Proprietor: ReCor Medical, Inc., Palo Alto, CA 94303 (US)
(72) Inventor: TAYLOR, Kevin, San Mateo, CA 94403 (US); CHANDLER, Paul, Santa Cruz, CA 95060 (US)
(74) Representative: Barker Brettell Sweden AB
(86) International application number: PCT/US2014/022796
(87) International publication number: WO 2014/159273

(56) References cited:
- EP-A2- 0 838 980
- WO-A2-2009/149315
- JP-A- 2000 054 153
- JP-A- 2001 111 126
- US-A- 3 775 816
- US-A- 3 938 502
- US-A- 5 456 259
- US-A1- 2003 138 571
- US-A1- 2006 088 705
- US-A1- 2007 167 764
- US-A1- 2009 204 006
- US-B2- 8 193 685

## Description

### BACKGROUND

### Field

This application relates generally to ultrasound transducers, and more specifically, to methods of plating a cylindrical base member comprising a piezoceramic material to produce the electrodes of the transducer.

### Description of the Related Art

Ultrasound transducers can comprise a ceramic base material having inner and/or outer electrodes that are electrically energized to produce acoustic energy. Therefore, methods of positioning the electrodes onto the surfaces of an ultrasound transducer are disclosed herein. US2007 167764 A1 describes a method for manufacturing a transducer component wherein the inner and outer walls of a cylindrical base member comprising a piezoceramic material are coated with a conductive material selected from copper, nickel, gold or silver.

### SUMMARY

The present invention is defined in the independent claims. Further embodiments of the invention are defined in the dependent claims.

The ultrasound transducer described herein comprises a cylindrical base member comprising a piezoceramic material, at least one layer of copper positioned along a surface of the cylindrical base member, at least one layer of nickel positioned along the at least one layer of copper, wherein the at least one layer of copper is positioned between the cylindrical base member and the at least one layer of nickel, and at least one layer of gold positioned along the least one layer of nickel. The copper, nickel and gold are deposited along exterior and interior surfaces of the cylindrical base member.

According to the invention, the base member comprises a piezoceramic material (e.g., PZT). In some embodiments, at least one electrode is deposited on a plurality of base members using a batch procedure (e.g., using a wire rack, a barrel system, etc.). In some embodiments, the base member is cut from a larger bulk member (e.g., a long ceramic cylinder).

The methods summarized above and set forth in further detail below describe certain actions taken by a practitioner (e.g., manufacturer); however, it should be understood that they can also include the instruction of those actions by another party. Thus, actions such as "depositing at least one layer" include "instructing depositing at least one layer."

### BRIEF DESCRIPTION OF THE DRAWINGS

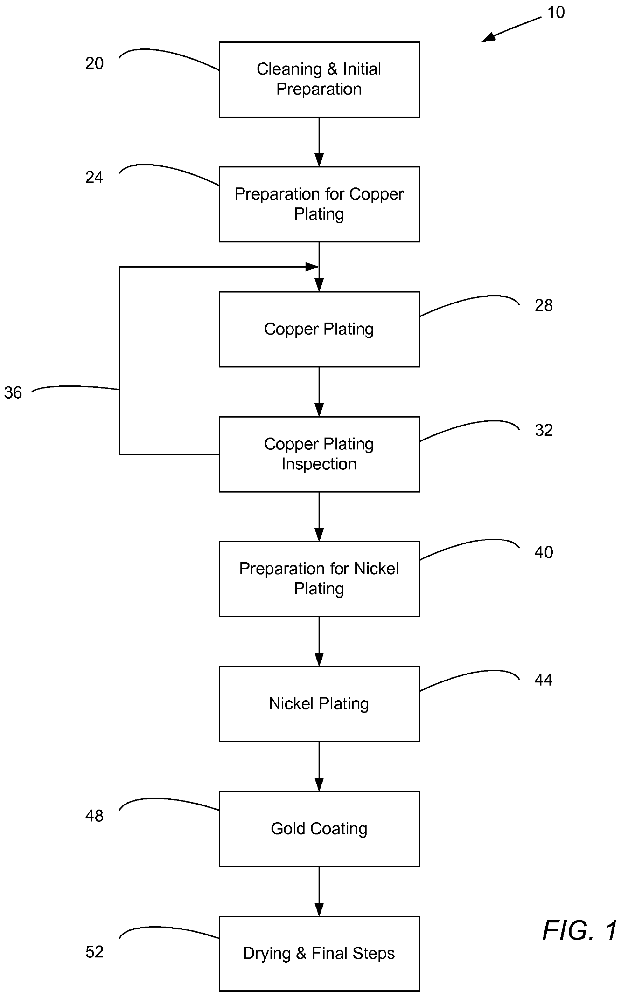

FIG. 1 schematically illustrates a flowchart of depositing an electrode on a base member of an ultrasound transducer according to one embodiment.

### DETAILED DESCRIPTION

According to the invention, ultrasound transducers include a cylindrical shape comprising a base material. Such a base material comprises lead zirconate titanate (PZT), other piezoelectric ceramic materials and/or the like. In some embodiments, a long cylindrical tube of ceramic base material is cut and/or otherwise machined into smaller sections to make individual transducers having a desired length.

One embodiment of a transducer plating method 10 is shown schematically in the flowchart of FIG. 1. In any of the embodiments disclosed herein, a plurality of the cylindrical tubes can be prepared and coated at the same time, e.g., in a batch system. For example, in some embodiments, a plurality of transducer tubes is positioned in a rack system (e.g., wire rack), a barrel system and/or the like. Accordingly, the multiple tubes can be simultaneously submerged, at least partially, into one or more baths or solutions during the preparation, plating and/or other manufacturing steps.

In examples not part of the invention, the various coating methods and techniques disclosed herein can be used on surfaces that are not cylindrical, such as, for example, flat surfaces, undulating surface, convex, concave or other rounded surfaces, irregularly-shaped surfaces, other non-cylindrical surface and/or the like. Accordingly, the various coating methods disclosed herein can be used to create electrodes for ultrasound transducers not forming part of the invention that are not cylindrical and/or transducers that does not have a cylindrical shape, such as, for example, flat transducers, concave or convex transducers, irregularly shaped transducers, etc.

### Cleaning and Initial Preparation

In some embodiments, machining oil, other oils, grease, natural coatings or layers and/or other materials are used to produce the individual tube lengths. Thus, it may be desirable or required to clean and otherwise prepare 20 the tubes before beginning the coating process. For example, in some embodiments, the process includes degreasing the tube using an ultrasonic degreaser, alcohol-based cleaner and/or any other cleaning product or agent. As noted above, the tubes can be submerged or otherwise placed within a bath (e.g., degreasing solution). After a particular time period of exposure to the degreaser or other cleaner (e.g., ~1 minute), the tubes can be removed and placed in a deionized water solution or bath (e.g., for ~1 minute) to remove excess degreaser and/or other cleaning solution. In other embodiments, the initial cleaning or degreasing of the surface on which an ultrasonic electrode will be placed can be accomplished using any other method or device, including, but not limited to, chemical cleaning, mechanical cleaning (e.g., sanding, resurfacing, etc.) and/or the like. In addition, the time period for the initial degreasing or other initial cleaning stage can be less than 1 minute (e.g., 0-20, 20-30, 30-40, 40-50, 50-60 seconds, time periods between the foregoing, etc.) or greater than 1 minute (e.g., I-IV2, I Vi-2, 2-3, 3-4, 4-5, 5-10 minutes, more than 10 minutes, etc.).

Next, in one embodiment, the surface of the tube can be further cleaned using an acid cleaner and/or other etching material. For example, the tube can be placed in a Citranox^{®} solution or other relatively weak acid solution for about 1 minute. In other embodiments, the time period for the acid cleaning or etching step or similar preparation step can be less than 1 minute (e.g., 0-20, 20-30, 30-40, 40-50, 50-60 seconds, time periods between the foregoing, etc.) or greater than 1 minute (e.g., I-IV2, I Vi-2, 2-3, 3-4, 4-5, 5-10 minutes, more than 10 minutes, etc.). This can help remove additional unwanted layers, coatings and/or materials from the exposed, exterior surfaces (e.g., inner and outer) of the tube. In some embodiments, an acid cleaning step (e.g., using a relatively weak acid) at least partially etches or resurfaces the outer surfaces of the tube. In some embodiments, a water rinse (e.g., using deionized water) or another fluid rinse can be used to remove excess acid cleaner from the tube.

### Preparation for Copper Plating

The tubes are initially plated with copper. One or more preparatory steps 24 can be taken, in some embodiments, in advance of the copper plating process. For example, the tube can be placed in a strong acid solution, such as, e.g., a 10% solution of HBF4 (e.g., tetrafluoroboric acid, other fluoroboric acid, etc.) and acetate. One or more other acids can be used, such as, for example, hydrochloric acid, sulfuric acid, nitric acid, any other acid, etc., can be used either in addition to or in lieu of HBF4 and acetate. In one embodiment, the tube is exposed to this solution for about 90 seconds (e.g., 60-120, 60-70, 70-80, 80-90, 90-100, 100-110, 110-120 seconds, time periods between the foregoing, etc.). In other embodiments, however, the exposure time during this step can be less or more than 90 seconds (e.g., 60-90, 90-120, 30-60 seconds, less than 30 seconds, more than 120 seconds, etc.), as desired or required by particular process (e.g., based on the amount of etching required, the type and strength of acid used, etc.). Regardless of the exact protocol, as a result of such an exposure, the tube's outer surfaces can be at least partially etched. This can help remove lead and/or other undesirable substances that may interfere with the subsequent copper plating steps. In some embodiments, care must be taken during the etching step to prevent damage to the geometry of the tube. In other words, if an excessive amount of etching is performed, the cylindrical shape of the tube can be changed, thereby negatively impacting the acoustic energy profile of the transducer. For example, if the tube is not cylindrical within a particular tolerance level, the acoustic energy emitted by the transducer may be unevenly delivered in the radial direction (e.g., creating hot spots, spots of lower energy intensity, etc.).

Once the tube has been adequately etched, it can be rinsed to remove any excess etching solution or material. For example, in one embodiment, the tube can be subjected to one, two or more deionized water rinses and/or other liquid-based rinses. For example, in some embodiments, the tubes are subjected to two separate deionized water rinse cycles, each of which can last about 30 seconds. In other embodiments, however, the rinse time can be less or more than 30 seconds (e.g., 0-10, 10-20, 20-30, 30-40, 40-50, 50-60 seconds, 1-2, 2-3, 3-4, 4-5 minutes, time periods between the foregoing, more than 5 minutes, etc.).

In some embodiments, the transducer tube is then subjected to a clean copper dummy load solution, e.g., a 10% solution of HBF4 (and/or another solution having a different acid, a different strength and/or other properties) for about 1 minute. In other embodiments, however, this step can be less or more than 1 minutes (e.g., 0-10, 10-20, 20-30, 30-40, 40-50, 50-60 seconds, 1-2, 2-3, 3-4, 4-5 minutes, time periods between the foregoing, more than 5 minutes, etc.). Such a step can help make the outer and inner surfaces of the tube more reactive for the subsequent copper plating step. In some embodiments, one or more sheets or other members comprising copper are positioned within a bath or solution into which the tube is placed. For example, in some embodiments, about ½ square foot of surface area of one or more copper-containing components (e.g., plates, other members, etc.) are placed in the bath about 1 to 5 minutes prior to starting the actual copper plating procedure.

Following its exposure to copper dummy load solution, the transducer tube can be rinsed during a water rinse stage. In some embodiments, the tube can be rinsed, for example, using deionized water for about 30 seconds. In other embodiments, however, the rinse time can be less or more than 30 seconds (e.g., 0-10, 10-20, 20-30, 30-40, 40-50, 50-60 seconds, 1-2, 2-3, 3-4, 4-5 minutes, time periods between the foregoing, more than 5 minutes, etc.).

### Copper Plating

With continued reference to FIG. 1, in some embodiments, following the copper plating preparation step, the transducer tube can proceed to a copper plating process 28. In one embodiment, the surfaces of the tube can be treated for the subsequent application of one or more plating catalysts. For example, the tube can be exposed to a sensitizer (e.g., Enthone 432) for about 1 minute. In some arrangements, the Enthone or other preparatory solution is exposed to one or more rinsing steps. For example, the tube can go through two rinsing steps using deionized water, each of which can last about 20 seconds. In other embodiments, however, the exposure time to the sensitizer and any subsequent rinsing steps can be shorter or longer than indicated above. For example, the tube can be exposed to the sensitizer for less or more than 1 minute (e.g., 0-10, 10-20, 20-30, 30-40, 40-50, 50-60 seconds, 1-2, 2-3, 3-4, 4-5 minutes, time periods between the foregoing, more than 5 minutes, etc.). Likewise, the subsequent rinsing can occur in a single step or multiple steps (e.g., 2, 3, 4, more than 4, etc.), and can last for less or more than 20 seconds (e.g., 0-5, 5-10, 10-15, 15-20, 20-25, 25-30, 30-40, 40-50, 50-60 seconds, 1-2, 2-3, 3-4, 4-5 minutes, time periods between the foregoing, more than 5 minutes, etc.).

Next, in some embodiments, the surfaces of the transducer tube can be catalyzed, at least in part, with palladium. For example, the tube can be placed in a bath of a catalytic solution (e.g., Enthone 440) for about 3 minutes. In some embodiments, the tube can be placed in contact with a catalytic solution for less or more than 3 minutes (e.g., 0-10, 10-20, 20-30, 30-40, 40-50, 50-60 seconds, 1-2, 2-3, 3-4, 4-5, 5-6, 6-7, 7-8, 8-9, 9-10 minutes, time periods between the foregoing, more than 10 minutes, etc.). In some embodiments, the palladium is a catalyst to assist in the subsequently plating of copper onto the tube surface. One or more other catalysts can be used in addition to palladium. Excess palladium-containing solution and/or other catalyst can then be removed using a quick dip procedure and/or any other procedure or step.

Once the surfaces of a transducer tube have been prepared, the tube can be placed in a bath (e.g., in a solution of Enthone 406, other copper-containing solution, etc.) to allow the copper to plate onto the tube. For example, in some embodiments, the tubes are kept in such a bath for about 10 minutes. In some embodiments, such a plating process can result in a copper coating on the tube of about 10-20 microinches (e.g., 10, 11, 12, 13, 14, 15, 16, 17, 18, 19, 20 microinches, thicknesses between the foregoing ranges, etc.). However, in some embodiments, the tube can be placed in a bath for this step for less or more than 10 minutes (e.g., 0-10, 10-20, 20-30, 30-40, 40-50, 50-60 seconds, 1-2, 2-3, 3-4, 4- 5, 5-6, 6-7, 7-8, 8-9, 9-10, 10-11, 11-12, 12-13, 13-14, 14-15, 15-20 minutes, time periods between the foregoing, more than 20 minutes, etc.). In addition, the resulting copper coating can be less than 10 microinches (e.g., 0-1, 1-2, 2-3, 3-4, 4-5, 5-6, 6-7, 7-8, 8-9, 9-10 microinches, etc.) or more than 20 microinches (e.g., 20-25, 25-30, 30-40, 40-50 microinches, dimensions between the foregoing, greater than 50 microinches, etc.), as desired or required.

### Copper Plating Inspection

In some embodiments, if, after an inspection phase 32, it is determined that the plating of the copper is inadequate (e.g., insufficient plating thickness, non-uniform plating, etc.), the tube can be exposed to one or more copper plating cycles 28. Thus, as schematically illustrated by step 36 in FIG. 1, the need to begin the plating process from the beginning (e.g., step 20 or 24 in FIG. 1) can be eliminated. This type of short-circuiting step 36 in the process 10 can reduce manufacturing time, simplify the manufacturing protocol and provide one or more benefits and advantages. In some embodiments, the short-circuiting step 36 can be repeated up to about 4 times before a transducer tube is discarded.

### Nickel Plating Preparation

In some embodiments, if the copper plating is satisfactory, the tube can be subsequently subjected to one or more nickel plating steps. In some embodiments, after the copper plating and inspection steps 28, 32, the tube can proceed to a nickel plating preparatory process 40. For example, the tube can be rinsed using a deionized water and/or other solution rinse for about 20 seconds before being exposed to an etching step. In some embodiments, the copper-plated tube can be at least partially etched in a 10% H2SO4 solution and/or any other acidic solution for about 30 seconds. In some embodiments, the rinsing and/or etching steps can be different than indicated above. For example, the tube can be rinsed for less or more than 20 seconds (e.g., 0-5, 5-10, 10-15, 15-20, 20-25, 30-40, 40-50, 50-60 seconds, 1-2, 2-3, 3-4, 4-5 minutes, time periods between the foregoing, more than 5 minutes, etc.). Further, the etching step can last for less or more than 30 seconds (e.g., 0-5, 5-10, 10-15, 15-20, 20-25, 30-40, 40-50, 50-60 seconds, 1-2, 2-3, 3-4, 4-5 minutes, time periods between the foregoing, more than 5 minutes, etc.), as desired or required. The use of H2SO4 and/or other acidic solution can provide, in some embodiments, a better match for the sulfate anion used in the previous copper plating steps, thereby facilitating the nickel plating process.

Once the copper-plated tube has been etched, it can be subjected to a palladium catalyst solution (e.g., TechniCatalyst AT 4000) and/or another catalyst solution or mixture. For example, the tube can be placed in a palladium catalyst solution for about 2 minutes. In some embodiments, the tube can be placed in the catalyst for less or more than 2 minutes (e.g., 0-5, 5-10, 10-15, 15-20, 20-25, 30-40, 40-50, 50-60 seconds, 1-2, 2-3, 3-4, 4-5 minutes, time periods between the foregoing, more than 5 minutes, etc.), as desired or required. In some embodiments, the palladium catalyst solution comprises a sulfate ion activator. In some embodiments, the palladium catalyst solution does not comprise a chloride ion activator. After exposure to the palladium catalyst, the copper dummy load can be terminated and the tube can be rinsed to remove any excess palladium catalyst solution (e.g., using deionized using a quick dip procedure).

### Nickel Plating

According to the invention, the transducer tube can then proceed to the nickel plating process 44. For example, the tube can be placed in a nickel solution for approximately 15 minutes. In some embodiments, the tube can be placed in a nickel solution for less or more than 15 minutes (e.g., 0-5, 5-10, 10-15, 15- 20, 20-25, 30-40, 40-50, 50-60 minutes, 1-2, 2-3, 3-4, 4-5 hours, time periods between the foregoing, more than 5 hours, etc.), as desired or required. In some embodiments, the nickel solution comprises a high-phosphorus nickel solution (e.g., NICHEM 5100). As a result of the exposure to such a nickel solution, in some embodiments, about 100-200 microinches (e.g., 150 microinches) of nickel can be electroplated onto the outside surface of the transducer tube (e.g., over the electroplated copper layer). After the nickel has been adequately plated on the outside surfaces of the transducer tube, excess nickel solution can be removed by rinsing the tube with deionized water (e.g., for about 20 seconds). In other embodiments, the rinsing step can last for less or more than 20 seconds (e.g., 0-5, 5-10, 10-15, 15-20, 20-25, 30-40, 40-50, 50-60 seconds, 1-2, 2-3, 3-4, 4-5 minutes, time periods between the foregoing, more than 5 minutes, etc.), as desired or required.

### Gold Immersion

According to the invention, a layer of gold is positioned 48 along the outside of the copper and nickel layers that have been plated on the transducer tube. For example, the gold can be immersed as a monolayer onto the outside of the tube. In other embodiments, more than one layer (e.g., 2, 3, more than 3, etc.) layers of gold are used, as desired or required. In some embodiments, the tube is subjected to an immersion of gold (e.g., OMG Fidelity 9027 + potassium gold) for about 2 minutes. The use of such an immersion layer can eliminate or reduce the likelihood of complications resulting from electrolytic plating of gold onto the surfaces of the cylinder, especially within the interior surfaces of relatively small cylinders. Therefore, in some embodiments, the gold is placed onto the transducer tube without using an electrolytic process. In some embodiments, the thickness of the gold monolayer deposited on the tube (e.g., along the outside of the copper and nickel layers) is about 2-10 microinches (e.g., 5 microinches). Following the gold immersion process, any excess gold can be removed from the outside of the tube using deionized water rinse (e.g., for about 20 seconds).

### Drying and Completion

According to some embodiments, after the desired layers of copper, nickel, gold and/or any other material have been placed along the outside of the transducer tube, the tube can undergo one or more finishing steps 52. For example, an alcohol rinse (comprising, e.g., isopropyl alcohol) can be used to remove any excess water and to facilitate drying of the outer surfaces of the tube. Finally, in some embodiments, the tube can be placed in an oven or other thermal environment to remove the alcohol and dry the tube.

A transducer tube plated in accordance with the various embodiments disclosed herein includes three different metals: a copper base layer, a nickel intermediate layer and a gold outer layer. In some embodiments, the thickness of the various metals placed on the tube can be about 150-200 microinches. For example, in one embodiment, a transducer can include a base layer of copper, an intermediate layer of nickel and an outer layer of gold having thicknesses of about 15 microinches, 150 microinches and 5 microinches, respectively. In other embodiments, the thickness of one or more layers can vary, as desired or required.

Additional details regarding possible ultrasonic transducer designs and embodiments (e.g., both structurally and operationally) are provided in U.S. Patent Application No. 11/267,123, filed on July 13, 2001 and published as U.S. Publ. No. 2002/0068885 on June 6, 2002; U.S. Patent Application No. 09/905,227, filed July 13, 2001 and issued as U.S. Patent No. 6,635,054 on October 21, 2003; U.S. Patent Application No. 09/904,620, filed on July 13, 2001 and issued as U.S. Patent No. 6,763,722 on July 20, 2004; U.S. Patent Application No. 10/783,310, filed February 20, 2004 and issued as U.S. Patent No. 7,837,676 on November 23, 2010; U.S. Patent Application No. 12/227,508, filed on February 3, 2010 and published as U.S. Publ. No. 2010/0130892 on May 27, 2010; U.S. Patent Application No. 10/611,838, filed on June 30, 2003 and published as U.S. Publ. No. 2004/0082859 on April 29, 2004; and PCT Appl. No. PCT/US2011/025543, filed on February 18, 2011 and published as PCT Publ. No. WO 2012/112165 on August 23, 2012.

To assist in the description of the disclosed embodiments, words such as upward, upper, bottom, downward, lower, rear, front, vertical, horizontal, upstream, downstream have been used above to describe different embodiments and/or the accompanying figures. It will be appreciated, however, that the different embodiments, whether illustrated or not, can be located and oriented in a variety of desired positions.

Language such as "up to," "at least," "greater than," "less than," "between," and the like includes the number recited. Numbers preceded by a term such as "about" or "approximately" include the recited numbers. For example, "about 10 mm" includes "10 mm." Terms or phrases preceded by a term such as "substantially" include the recited term or phrase. For example, "substantially parallel" includes "parallel."

## Claims

1. A method of depositing at least one electrode on a cylindrical base member of an ultrasound transducer, the method comprising:
at least partially etching a surface of the cylindrical base member using a first etching agent comprising an acid;
catalyzing the surface of the cylindrical base member using a first catalyst comprising palladium;
plating copper on the surface of the cylindrical base member;
at least partially etching a surface of the copper-plated surface using a second etching agent comprising an acid;
catalyzing the copper-plated surface using a second catalyst comprising palladium;
plating nickel on the copper-plated surface using an electroless plating process; and
depositing at least one layer of gold on the nickel-plated surface, wherein
the copper, nickel and gold are deposited along exterior and interior surfaces of the cylindrical base member; and
the cylindrical base member comprises a piezoceramic material.

2. The method of Claim 1, further comprising cleaning he cylindrical base member with a cleaning agent prior to at least partially etching the surface of the cylindrical base member using a the first etching agent.

3. The method of Claim 2, wherein the cleaning agent comprises at least one of a degreaser and an alcohol.

4. The method according to any one of the preceding claims, wherein plating copper on the surface of the cylindrical base member comprises placing the cylindrical base member in a copper bath.

5. The method according to any one of Claims 1 to 3, wherein plating copper on the surface of the cylindrical base member comprises using an electroless plating process.

6. An ultrasound transducer obtained by the method according to any one of Claims 1 to 5 comprising electrodes deposited on a cylindrical base member of the ultrasound transducer, wherein the ultrasound transducer comprises:
a cylindrical base member comprising a piezoceramic material;
at least one layer of copper positioned along a surfaces of the cylindrical base member;
at least one layer of nickel positioned along the at least one layer of copper, wherein the at least one layer of copper is positioned between the cylindrical base member and the at least one layer of nickel; and
at least one layer of gold positioned along the least one layer of nickel, wherein
the copper, nickel and gold are deposited along exterior and interior surfaces of the cylindrical base member.

7. The ultrasound transducer according to Claim 6, wherein a thickness of the at least one layer of copper is between 0.254 and 0.635 µm (10 and 25 microinches).

8. The ultrasound transducer according to Claim 7, wherein a thickness of the at least one layer of copper is 0.381 µm (15 microinches).

9. The ultrasound transducer according to Claim 6, wherein a thickness of the at least one layer of nickel is between 2.54 and 5.08 µm (100 and 200 microinches).

10. The ultrasound transducer according to Claim 9, wherein a thickness of the at least one layer of nickel is 3.81 µm (150 microinches).

11. The ultrasound transducer according to Claim 6, wherein a thickness of the at least one layer of gold is between 0.0025 and 0.254 µm (0.1 and 10 microinches).

12. The ultrasound transducer according to Claim 11, wherein a thickness of the at least one layer of gold is 0.127 µm (5 microinches).

13. The ultrasound transducer according to Claim 6, wherein a thickness of the at least one layer of copper is between 0.254 and 0.635 µm (10 and 25 microinches), a thickness of the at least one layer of nickel is between 2.54 and 5.08 µm (100 and 200 microinches), and a thickness of the at least one layer of gold is between 0.0025 and 0.254 µm (0.1 and 10 microinches).

## Patentansprüche

1. Verfahren zum Abscheiden mindestens einer Elektrode auf ein zylindrisches Basiselement eines Ultraschallwandlers, wobei das Verfahren umfasst:
zumindest teilweises Ätzen einer Oberfläche des zylindrischen Basiselements unter Verwendung eines ersten Ätzmittels, das eine Säure umfasst;
Katalysieren der Oberfläche des zylindrischen Basiselements unter Verwendung eines ersten Katalysators, der Palladium umfasst;
Verkupfern der Oberfläche des zylindrischen Basiselements;
zumindest teilweises Ätzen einer Oberfläche der verkupferten Oberfläche unter Verwendung eines zweiten Ätzmittels, das eine Säure umfasst;
Katalysieren der verkupferten Oberfläche unter Verwendung eines zweiten Katalysators, der Palladium umfasst;
Vernickeln kupferplattierten Oberfläche unter Verwendung eines stromlosen Vernickelungsprozesses; und
Abscheiden mindestens einer Schicht aus Gold auf die vernickelte Oberfläche, wobei
das Kupfer, Nickel und Gold entlang der Außen- und Innenflächen des zylindrischen Basiselements abgeschieden werden; und
das zylindrische Basiselement ein piezokeramisches Material umfasst.

2. Verfahren nach Anspruch 1, ferner umfassend Reinigen des zylindrischen Basiselements mit einem Reinigungsmittel vor dem zumindest teilweisen Ätzen der Oberfläche des zylindrischen Basiselements unter Verwendung des ersten Ätzmittels.

3. Verfahren nach Anspruch 2, wobei das Reinigungsmittel mindestens eines von einem Entfettungsmittel und einem Alkohol umfasst.

4. Verfahren nach einem der vorhergehenden Ansprüche, wobei das Verkupfern der Oberfläche des zylindrischen Basiselements das Platzieren des zylindrischen Basiselements in einem Kupferbad umfasst.

5. Verfahren nach einem der Ansprüche 1 bis 3, wobei das Verkupfern der Oberfläche des zylindrischen Basiselements das Verwenden eines stromlosen Verkupferungsprozesses umfasst.

6. Ultraschallwandler, der durch das Verfahren nach einem der Ansprüche 1 bis 5 erhalten wird, umfassend Elektroden, die auf einem zylindrischen Basiselement des Ultraschallwandlers abgeschieden werden, wobei der Ultraschallwandler umfasst:
ein zylindrisches Basiselement, das ein piezokeramisches Material umfasst;
mindestens eine Kupferschicht, die entlang einer Oberflächen des zylindrischen Basiselements positioniert ist;
mindestens eine Nickelschicht, die entlang der mindestens einen Kupferschicht positioniert ist, wobei die mindestens eine Kupferschicht zwischen dem zylindrischen Basiselement und der mindestens einen Nickelschicht positioniert ist; und
mindestens eine Goldschicht, die entlang der mindestens einen Nickelschicht positioniert ist, wobei
das Kupfer, Nickel und Gold entlang der Außen- und Innenflächen des zylindrischen Basiselements abgeschieden werden.

7. Ultraschallwandler nach Anspruch 6, wobei eine Dicke der mindestens einen Schicht aus Kupfer zwischen 0,254 und 0,635 µm (10 und 25 Mikrozoll) beträgt.

8. Ultraschallwandler nach Anspruch 7, wobei eine Dicke der mindestens einen Schicht aus Kupfer 0,381 µm (15 Mikrozoll) beträgt.

9. Ultraschallwandler nach Anspruch 6, wobei eine Dicke der mindestens einen Schicht aus Nickel zwischen 2,54 und 5,08 µm (100 und 200 Mikrozoll) beträgt.

10. Ultraschallwandler nach Anspruch 9, wobei eine Dicke der mindestens einen Schicht aus Nickel 3,81 µm (150 Mikrozoll) beträgt.

11. Ultraschallwandler nach Anspruch 6, wobei eine Dicke der mindestens einen Schicht aus Gold zwischen 0,0025 und 0,254 µm (0,1 und 10 Mikrozoll) beträgt.

12. Ultraschallwandler nach Anspruch 11, wobei eine Dicke der mindestens einen Schicht aus Gold 0,127 µm (5 Mikrozoll) beträgt.

13. Ultraschallwandler nach Anspruch 6, wobei eine Dicke der mindestens einen Schicht aus Kupfer zwischen 0,254 und 0,635 µm (10 und 25 Mikrozoll) beträgt, eine Dicke der mindestens einen Schicht aus Nickel zwischen 2,54 und 5,08 µm (100 und 200 Mikrozoll) beträgt und eine Dicke der mindestens einen Schicht aus Gold zwischen 0,0025 und 0,254 µm (0,1 und 10 Mikrozoll) beträgt.

## Revendications

1. Procédé de dépôt d'au moins une électrode sur un élément de base cylindrique d'un capteur ultrasonore, le procédé comprenant :
la gravure d'au moins une partie de la surface de l'élément de base cylindrique au moyen d'un premier agent de gravure comprenant un acide ;
la catalyse de la surface de l'élément de base cylindrique au moyen d'un premier catalyseur comprenant du palladium ;
le placage de cuivre sur la surface de l'élément de base cylindrique ;
la gravure d'au moins une partie de la surface plaquée de cuivre au moyen d'un deuxième agent de gravure comprenant un acide ;
la catalyse de la surface plaquée de cuivre au moyen d'un deuxième catalyseur comprenant du palladium ;
le placage de nickel sur la surface plaquée de cuivre au moyen d'un processus de placage autocatalytique ; et
le dépôt d'au moins une couche d'or sur la surface plaquée de nickel, dans lequel
le cuivre, le nickel et l'or sont déposés le long de surfaces extérieure et intérieure de l'élément de base cylindrique ; et
l'élément de base cylindrique comprend un matériau piézocéramique.

2. Procédé selon la revendication 1, comprenant en outre le nettoyage de l'élément de base cylindrique avec un agent de nettoyage avant la gravure d'au moins une partie de la surface de l'élément de base cylindrique au moyen du premier agent de gravure.

3. Procédé selon la revendication 2, dans lequel l'agent de nettoyage comprend au moins un parmi un dégraissant et un alcool.

4. Procédé selon l'une quelconque des revendications précédentes, dans lequel le placage de cuivre sur la surface de l'élément de base cylindrique comprend le placement de l'élément de base cylindrique dans un bain de cuivre.

5. Procédé selon l'une quelconque des revendications 1 à 3, dans lequel le placage de cuivre sur la surface de l'élément de base cylindrique comprend l'utilisation d'un processus de placage autocatalytique.

6. Capteur ultrasonore obtenu par le procédé selon l'une quelconque des revendications 1 à 5 comprenant des électrodes déposées sur un élément de base cylindrique du capteur ultrasonore, dans lequel le capteur ultrasonore comprend :
un élément de base cylindrique comprenant un matériau piézocéramique ;
au moins une couche d'or positionnée le long de l'au moins une couche de nickel, dans lequel
au moins une couche de nickel positionnée le long de l'au moins une couche de cuivre, dans lequel l'au moins une couche de cuivre est positionnée entre l'élément de base cylindrique et l'au moins une couche de nickel ; et
au moins une couche d'or positionnée le long de l'au moins une couche de nickel, dans lequel
le cuivre, le nickel et l'or sont déposés le long de surfaces extérieure et intérieure de l'élément de base cylindrique.

7. Capteur ultrasonore selon la revendication 6, dans lequel une épaisseur de l'au moins une couche de cuivre est comprise entre 0,254 et 0,635 µm (10 et 25 micropouces).

8. Capteur ultrasonore selon la revendication 7, dans lequel une épaisseur de l'au moins une couche de cuivre est de 0,381 µm (15 micropouces).

9. Capteur ultrasonore selon la revendication 6, dans lequel une épaisseur de l'au moins une couche de nickel est comprise entre 2,54 et 5,08 µm (100 et 200 micropouces).

10. Capteur ultrasonore selon la revendication 9, dans lequel une épaisseur de l'au moins une couche de nickel est de 3,81 µm (150 micropouces).

11. Capteur ultrasonore selon la revendication 6, dans lequel une épaisseur de l'au moins une couche d'or est comprise entre 0,0025 et 0,254 µm (0,1 et 10 micropouces).

12. Capteur ultrasonore selon la revendication 11, dans lequel une épaisseur de l'au moins une couche d'or est de 0,127 µm (5 micropouces).

13. Capteur ultrasonore selon la revendication 6, dans lequel une épaisseur de l'au moins une couche de cuivre est comprise entre 0,254 et 0,635 µm (10 et 25 micropouces), une épaisseur de l'au moins une couche de nickel est comprise entre 2,54 et 5,08 µm (100 et 200 micropouces), et une épaisseur de l'au moins une couche d'or est comprise entre 0,0025 et 0,254 µm (0,1 et 10 micropouces).
